(19) Europäisches Patentamt
European Patent Office
Office européen des brevets

(11) **EP 3 142 738 B1**

(12) **EUROPEAN PATENT SPECIFICATION**

(45) Date of publication and mention
of the grant of the patent:
**04.11.2020 Bulletin 2020/45**

(21) Application number: **15722638.2**

(22) Date of filing: **04.05.2015**

(51) Int Cl.:
*B29C 57/00* (2006.01)    *B29L 31/00* (2006.01)
*A61M 25/10* (2013.01)    *A61M 25/00* (2006.01)
*B29C 55/22* (2006.01)

(86) International application number:
**PCT/US2015/029041**

(87) International publication number:
**WO 2015/175252 (19.11.2015 Gazette 2015/46)**

(54) **HIGH PRESSURE LOW COST MULTILAYER BALLOON CATHETER**

KOSTENGÜNSTIGER MEHRSCHICHTIGER HOCHDRUCK-BALLONKATHETER

CATHÉTER À BALLONNET MULTICOUCHE À FAIBLE COÛT À HAUTE PRESSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2014 US 201461994437 P**

(43) Date of publication of application:
**22.03.2017 Bulletin 2017/12**

(73) Proprietor: **Boston Scientific Scimed Inc.**
**Maple Grove, Minnesota 55311 (US)**

(72) Inventors:
• **STROUD, Shannon Rodney**
**Osseo, Minnesota 55369 (US)**
• **SQUIRE, Robert N.**
**Maple Grove, Minnesota 55369 (US)**
• **HORN, Daniel James**
**Shoreview, Minnesota 55126 (US)**
• **ROYER, Adam Joseph**
**Brooklyn Park, Minnesota 55444 (US)**

(74) Representative: **Vossius & Partner**
**Patentanwälte Rechtsanwälte mbB**
**Siebertstrasse 3**
**81675 München (DE)**

(56) References cited:
**EP-A1- 0 921 832       EP-A1- 1 100 573**
**US-A1- 2009 198 266    US-A1- 2010 130 926**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 142 738 B1

**Description**

BACKGROUND OF THE DISCLOSURE

Balloon catheters are employed in a variety of medical procedures including plain old balloon angioplasty (POBA) as well as for delivery of medical devices to the treatment site such as stent delivery.

Medical applications where a balloon catheter is employed intraluminally such as for POBA and stent delivery can be demanding applications due to the extremely small vessels, and the tortuous and long distances the catheter may travel to the treatment site, For this reason, trackability is an important parameter for a balloon catheter. Also, for the balloon, it is typically desirable that the balloon be thin walled and have high strength.

The art referred to and/or described above is not intended to constitute an admission that any patent, publication or other information referred to herein is "prior art" with respect to the present disclosure.

US 2010/130926 A1 relates to a balloon catheter, comprising a shaft having a proximal end, a distal end, and an inflation lumen extending therein, and a balloon on the shaft which has an interior in fluid communication with the inflation lumen. The balloon has an outer layer and an inner layer. The outer layer comprises a material having a first transition temperature, and the inner layer comprises a material having a second transition temperature. The second transition temperature is higher than the first transition temperature and the balloon is formed at a temperature between said first and second transition temperatures.

US 2009/198266 A1 relates to a method, comprising expanding a first balloon distally of a gastro-esophageal junction; and contacting the first balloon to a proximal portion of a stomach.

EP 1100573 A1 relates to a method of making dilatation balloons with reduced cone stiffness. The method includes a step of drawing material from a cone section after the parison has been inflated to conform to a mold cavity configuration. The method may utilize a mold whose cavity includes arcuate walls defining the balloon's end cones and a predetermined minimal distance from the side edges of the mold to the points where the arcuate walls intersect with a smaller diameter balloon stem portion.

EP 0921832 A1 relates to a method of forming a balloon for a medical device, wherein a tubing of a thermoplastic polymer material is radially expanded under a first pressure at a first temperature to form the balloon at a first diameter. The method further includes the step of annealing the balloon at a second temperature above ambient and less than the first temperature and a second pressure for a time sufficient to shrink the formed balloon to a second diameter less than the first diameter. The method also includes the step of pressurizing the balloon in a fixed diameter form at a certain pressure level and at a temperature level not less than said second temperature for a time. The resulting diameter of the balloon is controlled by performing the above-mentioned steps.

Without limiting the scope of the invention a brief summary of some of the claimed embodiments of the present disclosure is set forth below. Additional details of the summarized embodiments of the invention and/or additional embodiments of the present disclosure may be found in the Detailed Description of the Invention below.

BRIEF SUMMARY OF THE DISCLOSURE

In one aspect, the present disclosure relates to a multilayer shaft with an integral balloon having a total shaft length of at least 70 cm.

The integral balloon may have a burst strength of at least 30,000 psi (3.068427x$10^8$ Pa).

The integral balloon may have a distension per atmosphere between 60.7950*$10^4$ and 1.419*$10^6$ N/m$^2$ (6 and 14 atmospheres) that is no greater than 0.9%.

The integral balloon may have a double wall thickness of 0.0254 mm to 0.127 mm.

The integral balloon may have a balloon outer diameter of 1.5 mm to 28 mm.

The shaft may have a shaft outer diameter that is 15-55% of the balloon outer diameter.

The shaft may have a shaft inner diameter that is 11-50% of the balloon outer diameter.

The shaft may have an outer layer formed of nylon and an inner layer formed of poly(ether-block-amide).

The shaft may have an inner layer formed of poly(ether-block-amide), a middle layer formed of nylon, and an outer layer formed of poly(ether-block-amide).

The nylon may be selected from the group consisting of aromatic and aliphatic nylons. The aliphatic nylons may be selected from the group consisting of nylon 12; nylon 6; nylon 6/10; nylon 6/12; and nylon 11.

The poly(ether-block-amide) may have a shore D hardness of 60-74D.

In a further aspect, the present disclosure relates to a tubular shaft comprising a wall that comprises: an outer layer of poly(ether-block-amide); a middle layer of nylon; an inner layer of poly(ether-block-amide); wherein a section of the wall is expandable or inflatable.

The distal end of the section of the wall may be proximal to the distal end of the tubular shaft.

The section of the wall may be a balloon.

The section of the wall may have a double wall thickness of about 0.0254 mm to about 0.127 mm.

The section of the wall may have a burst strength of at least 30,000 psi ($3.068427 \times 10^8$ Pa).

The section of the wall may have a distension per atmosphere between $60.7950 \times 10^4$ and $1.419 \times 10^6$ N/m$^2$ (6 and 14 atmosphere) that is no greater than 0.9%.

The section of the wall may have an outer diameter of about 1.5 mm to about 28 mm.

The tubular shaft may have a length of at least 70 cm.

The remainder of the tubular shaft may have an outer diameter of 15-55% of the outer diameter of the section of the wall.

The remainder of the tubular shaft may have an inner diameter of 11-5 0% of the outer diameter of the section of the wall.

The inner and outer layers may be formed of poly(ether-block-amide) having a shore D of about 60-74. The outer layer may have a lower shore D than the inner layer.

The nylon of the inner layer may be selected from the group consisting of aromatic and aliphatic nylons. The aliphatic nylons may be selected from the group consisting of nylon 12; nylon 6; nylon 6/10; nylon 6/12; and nylon 11.

The tubular shaft may define a lumen. The lumen may be an inflation lumen. The lumen may be a single lumen.

The tubular shaft may form a part of a balloon catheter.

The balloon catheter may include an inner shaft positioned inside the tubular shaft. A distal end region of the inner shaft may be secured to a distal end region of the tubular shaft. The inner shaft of the balloon catheter may define a guidewire lumen.

The balloon catheter may further include a manifold at a proximal end. The manifold may be secured to the tubular shaft and the inner shaft. The manifold may form the proximal end of the balloon catheter. The manifold may include a plurality of outlets. The plurality of outlets may include a first outlet in communication with the lumen defined by the tubular shaft and a second outlet in communication with the guidewire lumen.

The balloon catheter may consist of the tubular shaft, the inner shaft positioned inside the tubular shaft, and the manifold.

In another aspect, the present disclosure relates to a method of forming the multilayer shaft where the method comprises: a first stretching step wherein a portion of a distal end region of a multilayer polymeric tube is stretched at a first pressure and a first temperature, and the first pressure is equal to ambient pressure; a second stretching step wherein the multilayer polymeric tube is stretched at a second pressure and the first temperature until the extruded tube is stretched to 25 to 75%, preferably, 35-65%, more preferably 45-55%, and most preferably 50% the desired final length, and the second pressure is greater than the first pressure; a third stretching step wherein the multilayer polymeric tube is stretched at a third pressure and the first temperature to form a stretched multilayer polymeric tube having a final stretched length, and the third pressure is less than the second pressure and greater than the first pressure; and a balloon forming step wherein a section of the stretched multilayer polymeric tube is formed into an integral balloon.

In a further aspect of the method, forming the integral balloon may comprise: placing the section of the stretched multilayer polymeric tube into a balloon mold; pressurizing the stretched multilayer polymeric tube to a fourth pressure at a second temperature to form the section into the integral balloon, wherein the fourth pressure is different than the first, second, and third pressures, and the second temperature is greater than the first temperature; and heat setting the integral balloon at a third temperature greater than the second temperature.

The method may further comprise a first quenching step after the third stretching step, wherein during the first quenching step the temperature is reduced to a fourth temperature less than the first temperature and pressure is reduced from the third pressure.

The method may further comprise a second quenching step after the heat setting step, wherein during the second quenching step the temperature is reduced to a fifth temperature less than the first temperature. The integral balloon may be removed from the mold after the second quenching step.

In a further aspect of the method, the multilayer polymeric tube may be formed by coextrusion.

In a further aspect of the method, the multilayer polymeric tube may include a layer of poly(ether-block-amide) and a layer of nylon.

In a further aspect of the method, the multilayer polymeric tube may include a layer of poly(ether-block-amide) and a layer of nylon. The poly(ether-block-amide) may have a shore D hardness of about 60-74D. The nylon may be selected from the group consisting of aromatic and aliphatic nylons. The aliphatic nylons may be sclcctcd from the group consisting of nylon 12; nylon 6; nylon 6/10; nylon 6/12; and nylon 11.

In a further aspect, the present disclosure relates to a method of making a balloon catheter comprising: inserting an inner shaft into a lumen of the multilayer shaft; and securing a distal end region of the inner shaft to a distal end region of the multilayer shaft.

A hot jaw, a laser bonder, a means of fusion, or a means of adhesion may be used to secure the distal end region of the inner shaft to the distal end region of the multilayer shaft.

The method of making a balloon catheter may further comprise attaching a manifold to the inner shaft and the multilayer shaft. The manifold may be attached by an adhesive selected from the group consisting of: a UV cure adhesive; a two part epoxy; and a high strength adhesive.

## BRIEF SUMMARY OF THE INVENTION

In accordance with the present invention, there is provided a method of forming a multilayer shaft as defined in claim 1.

In addition, further advantageous embodiments follow from the dependent claims.

References to "embodiments" throughout the description which are not under the scope of the appended claims merely represent possible exemplary executions and are therefore not part of the present invention. The invention is defined by the appended claims.

These and other aspects of an integral balloon shaft, a balloon catheter, methods of making an integral balloon shaft, and methods of making the balloon catheter are pointed out with particularity in the claims annexed hereto and forming a part hereof. However, for further understanding reference can be made to the drawings which form a further part hereof and the accompanying descriptive matter, in which one or more embodiments are illustrated and described.

## BRIEF DESCRIPTION OF THE SEVERAL VIEWS OF THE DRAWING(S)

FIG. 1 is a view of a balloon catheter.
FIG. 2 is an enlarged view of a portion of the balloon catheter of FIG. 1.
FIG. 3 is a graft of the experimental results for the average balloon burst pressure.
FIG. 4 is a graft of the experimental results for the average double wall thickness.

## DETAILED DESCRIPTION OF THE INVENTION

While the subject matter of the present disclosure may be embodied in many different forms, there are described in detail herein specific preferred embodiments of the present disclosure. This description is an exemplification of the principles of the present disclosure and is not intended to limit the present disclosure to the particular embodiments illustrated.

For the purposes of this disclosure, like reference numerals in the figures shall refer to like features unless otherwise indicated.

As used in this disclosure, the terms "connect"; "engage"; "secure"; "attach" do not include "indirect" connection, engagement, securement or attachment. Thus, for example Element B "connecting" Elements A and C, directly connects A and C with no other element between A and B or between B and C.

As used in this disclosure, an "end" is the last part or extremity of an element, while an "end region" of an element is a region adjacent to, and includes, the "end."

As used in this disclosure, a "section" extends from a first longitudinal position to a second longitudinal position of the tubular shaft or wall, and extends around the entire circumference of the tubular shaft or wall.

### Integral Balloon Shaft

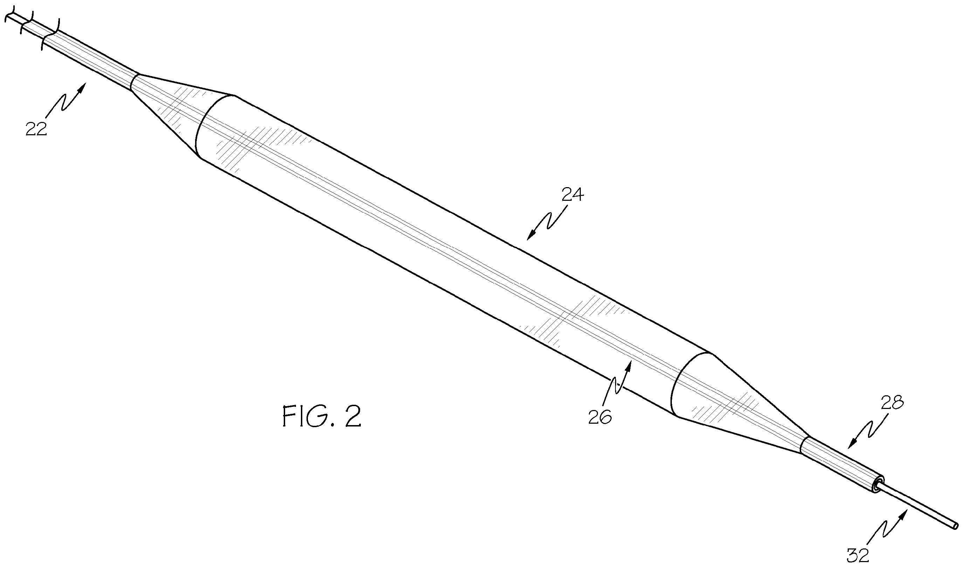

The integral balloon shaft 22 of the present disclosure is a tubular shaft 22 with an integral balloon 24 (e.g. FIG. 2). As used in this disclosure, "integral" means one-piece construction. In one aspect, the balloon shaft 22 has a length of at least 70 cm and defines a single lumen that is an inflation lumen. The integral balloon shaft 22 has one or more of the following attributes: a balloon 24 with: a burst strength of at least 30,000 psi ($2.068427 \times 10^8$ Pa); a burst pressure of at least 280 psi ($1.930532 \times 10^6$ Pa); a double wall thickness of about 0.001 inches to about 0.005 inches (about 0.0254 mm to about 0.127 mm); a distension per atmosphere between $60.7950 \times 10^4$ and $1.419 \times 10^6$ N/m$^2$ (6 and 14 atmosphere) that is no greater than 0.9%; a balloon outer diameter of about 1.5 to about 28 mm; and a shaft 22 with a shaft outer diameter (OD) that is 15-55% of the balloon outer diameter (OD); and a shaft inner diameter (ID) that is 11-50% of the balloon outer diameter (OD).

The balloon shaft 22 may be formed from an extruded multilayer polymeric tube so that the balloon shaft 22 has at least two polymeric layers forming a wall of the balloon shaft 22. Thus, as used herein, a "multilayer shaft" has no gaps between adjacent layers forming the balloon shaft 22.

Suitable polymers for the polymeric layers include, but are not limited to: poly(ether-block-amides) and nylons. In one aspect, the balloon shaft 22 has two polymeric layers, an outer polymeric layer and an inner polymeric layer. In a further aspect of a balloon shaft 22 with two polymeric layers, nylon forms the outer polymeric layer and poly(ether-block-amide) forms the inner polymeric layer.

For example, the balloon shaft 22 may have three polymeric layers, an outer polymeric layer, a middle polymeric layer, and an inner polymeric layer. The outer polymeric layer may have a lower shore D hardness than the inner polymeric layer. At least one of the polymeric layers may comprise nylon. An outer shaft with three polymeric layers, a poly(ether-block-amide) may form the outer and inner layers and a nylon forms the middle layer.

The poly(ether-block-amides) may have a shore D hardness of about 60-74D. Poly(ether-block-amide) copolymers are available from Arkema, North America under the tradename of Pebax®. Arkema's headquarters are located in Philadelphia, Pa. Specific grades of Pebax® useful herein include, but are not limited to 6333, 7033, and 7233.

Suitable nylons include aromatic and aliphatic nylons. Examples of aliphatic nylons include nylon 12, nylon 6, nylon 6/10, nylon 6/12 and nylon 11. Nylon 12 is available from a variety of polymer manufacturers. For example, nylon 12 is available from Degussa-Hüls AG, North America under the tradename Vestamid® L2101F. Degussa's national headquarters are located in Düsseldorf, Germany. Examples of aromatic nylons include the Grivory family of polymers (commercially available from EMS (Sumter, S.C.)), nylon MXD-6 polymers (commercially available from Mitsubishi Gas Chemical (Tokyo, Japan)), and the Trogamid family of polymers (commercially available from Degussa AG (Germany). The nylon may have a shore D hardness of 40-88.

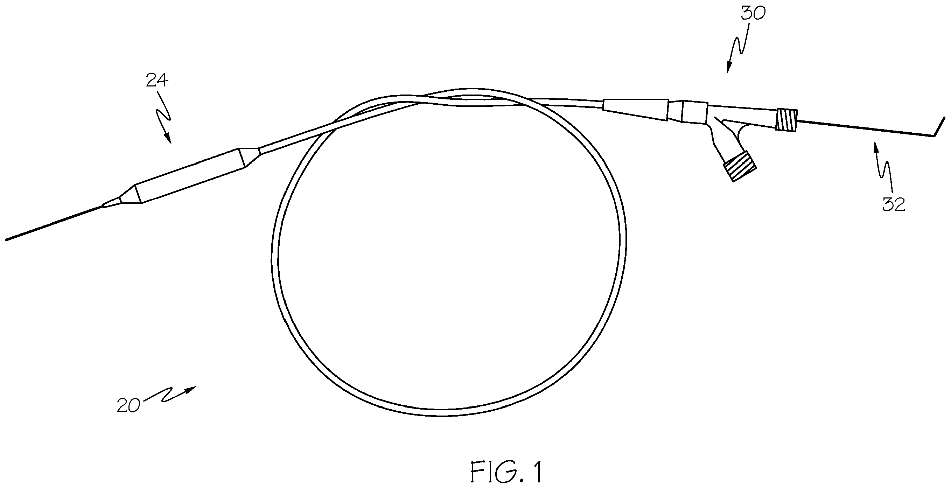

A section 24 of the balloon shaft 22 is inflatable or expandable (e.g. FIGS. 1-2). Since the section 24 of the balloon shaft 22 is inflatable or expandable, the section 24 can be described as a balloon, the inflatable section 24 of the balloon shaft 22 is referred to herein as a balloon 24. Since the balloon 24 is a part of the balloon shaft 22, the balloon 24 is not bonded or welded to the balloon shaft 22. Therefore since the balloon 24 is not a separate component secured to the balloon shaft 22, the balloon 24 and balloon shaft 22 can be described as: a single component; a one-piece construction; or integral. Further, since the balloon 24 is a part of the balloon shaft 22 at least one of the polymeric layers of the balloon 24 is the same as the polymeric layers of the balloon shaft 22. Typically, all layers of the balloon 24 are the same as the layers of the balloon shaft 22.

The balloon 24 can have any suitable longitudinal length and diameter. As noted above, one characteristic of the integral balloon 24 is a burst strength of at least 30,000 psi ($2.068427 \times 10^8$ Pa). Burst strength, a measure of the strength of the balloon, is calculated using the following formula:

$$\text{Strength (psi)} = PxD/2t$$

where P = internal pressure when the balloon bursts, the burst pressure (psi) (1 psi = 6894.75729 Pa); D is the exterior diameter of the balloon (cm); and t is the wall thickness of the portion of the balloon with the larger exterior diameter (cm).

Examples of a balloon shaft 22 with an integral balloon 24 are provided in Table 1. Reference to Examples A-E will be made hereinafter.

**Table 1**

| Ex. | Layers | Balloon | |
| | | Length (mm) | Diameter (mm) |
| --- | --- | --- | --- |
| A | 3 | 40 | 3 |
| B | 2 | 40 | 7 |
| C | 3 | 40 | 7 |
| D | 3 | 40 | 7 |
| E | 3 | 40 | 12 |

The trilayer balloon shafts 22 of Examples A and C-E have an outer layer of poly(ether-block-amide); a middle layer of nylon; and an inner layer of poly(ether-block-amide). The bilayer balloon shaft 22 of Example B has an outer layer of nylon and an inner layer of poly(ether-block-amide).

In one aspect, the integral balloon shaft 22 is formed from a multilayer polymeric tube (hereinafter extruded tube). Each layer of the extruded tube extends from one end of the extruded tube to the other end of the extruded tube. Any suitable means can be used to form the multilayer polymeric tube. In one aspect, the multilayer polymeric tube is formed by coextrusion.

Some exemplary inner and outer diameters for an extruded tube are provided in Table 2. A portion of the end section is formed into the balloon 24.

**Table 2**

| | | Extruded Tube Dimensions | | | |
|---|---|---|---|---|---|
| | | Tube End Section | | Remainder of Tube | |
| Ex. | OD | ID | OD | ID | |
| A | 0.0391 in 0.99314 mm | 0.0213 in 0.54102 mm | 0.0660 in 1.67640 mm | 0.0470 in 1.19380 mm | |
| B | 0.0686 in 1.74244 mm | 0.0437 in 1.10998 mm | 0.0686 in 1.74244 mm | 0.0437 in 1.10998 mm | |
| C | 0.0689 in 1.75006 mm | 0.0452 in 1.14808 mm | 0.0689 in 1.75006 mm | 0.0452 in 1.14808 mm | |
| D | 0.0714 in 1.81356 mm | 0.0452 in 1.14808 mm | 0.0714 in 1.81356 mm | 0.0452 in 1.14808 mm | |
| E | 0.1094 in 2.77876 mm | 0.0706 in 1.79324 mm | 0.1094 in 2.77876 mm | 0.0706 in 1.79324 mm | |

Aspects of the balloon of Examples A-E are provided in Table 3 where 2x wall is a measurement of the double wall thickness; D is the diameter of the balloon; burst pressure is the pressure at which the balloon bursts; and distension (Dist.) is a measure of percent balloon expansion per $10.1325 \times 10^4$ N/m$^2$ (atmosphere) between $60.7950 \times 10^4$ and $1.419 \times 10^6$ N/m$^2$ (6 and 14 atm). The measurement of the double wall thickness (2x wall) can be obtained by measuring the thickness of a deflated flattened balloon. Distension of the balloon is measured between $60.7950 \times 10^4$ and $1.419 \times 10^6$ N/m$^2$ (6 and 14 atmospheres) by inflating the balloon initially to $60.7950 \times 10^4$ N/m$^2$ (6 atmospheres) and measuring the expansion of the balloon as the balloon is further inflated to $1.419 \times 10^6$ N/m$^2$ (14 atmospheres). As noted above, the balloon has a distension per atmosphere between $60.7950 \times 10^4$ and $1.419 \times 10^6$ N(m$^2$ (6 and 14 atmospheres) that is no greater than 0.9%. Thus, when the balloon is inflated from $60.7950 \times 10^4$ to $7.09275 \times 10^5$ N/m$^2$ (6-7 atmospheres), $7.09275 \times 10^5$ to $8.10600 \times 10^5$ N/m$^2$ (7-8 atmospheres), $8.10600 \times 10^5$ to $9.11925 \times 10^5$ N/m$^2$ (8-9 atmospheres), $9.11925 \times 10^5$ to $1.013 \times 10^6$ N/m$^2$ (9-10, atmospheres), $1.013 \times 10^6$ to $1.115 \times 10^6$ N/m$^2$ (10-11 atmospheres), $1.115 \times 10^6$ to $1.216 \times 10^6$ N/m$^2$ (11-12 atmospheres), $1.216 \times 10^6$ to $1.317 \times 10^6$ N/m$^2$ (12-13 atmospheres), or $1.317 \times 10^6$ to $1.419 \times 10^6$ N/m$^2$ (13-14 atmospheres), the balloon expands/distends no more than 0.9%.

**Table 3**

| | | | Balloon Properties | | | |
|---|---|---|---|---|---|---|
| Ex. | Avg. 2x wall | Min. 2x wall | D at $60.7950 \times 10^4$ N/m$^2$ [6 atm] (mm) | Burst Pressure | Dist. | Burst Strength |
| A | 0.00178 in 0.045212 mm | 0.00168 in 0.042672 mm | 3.14 | 454 psi $3.13 \times 10^6$ Pa | 0.73 | 33359 psi $2.30 \times 10^8$ Pa |
| B | 0.00217 in 0.055118 mm | 0.00210 in 0.05334 mm | 7.04 | 367 psi $2.53 \times 10^6$ Pa | 0.59 | 48529 psi $3.35 \times 10^8$ Pa |
| C | 0.00203 in 0.051562 mm | 0.00192 in 0.048768 mm | 7.17 | 309 psi $2.13 \times 10^6$ Pa | 0.71 | 45332 psi $3.13 \times 10^8$ Pa |
| D | 0.00236 in 0.059944 mm | 0.00232 in 0.058928 mm | 7.13 | 353 psi $2.43 \times 10^6$ Pa | 0.68 | 42696 psi $2.94 \times 10^8$ Pa |
| E | 0.00314 in 0.079756 mm | 0.00307 in 0.077978 mm | 11.84 | 289 psi $1.99 \times 10^6$ Pa | 0.51 | 43853 psi $3.02 \times 10^8$ Pa |

Aspects of the integral balloon shaft 22 of Examples A-E are provided in Table 4.

**Table 4**

| Ex. | Integral Balloon Shaft | | | |
|---|---|---|---|---|
| | OD: Shaft /Balloon | ID: Shaft /Balloon | Shaft Wall/Balloon Wall | Shaft ID % strain |
| A | 0.53 | 0.47 | 10.674 | 23% |
| B | 0.25 | 0.22 | 11.475 | 37% |
| C | 0.24 | 0.21 | 11.675 | 33% |
| D | 0.25 | 0.21 | 11.102 | 33% |
| E | 0.23 | 0.14 | 12.357 | -8% |

As noted above, the shaft 22 may have a shaft outer diameter (OD) that is 15-55% of the balloon outer diameter (OD); and a shaft inner diameter (ID) that is 11-50% of the balloon outer diameter (OD). As can be seen in Table 4, the comparative outer diameter and inner diameter measurements depend in part on the size of the balloon. Thus, for example, a 3 mm balloon may have a shaft outer diameter (OD) that is 40-55% of the balloon outer diameter (OD); a 3mm balloon may have a shaft inner diameter (ID) that is 35 to 50% of the balloon outer diameter; a 7 mm balloon may have a shaft outer diameter (OD) that is 20-35% of the balloon outer diameter (OD); a 7 mm balloon may have a shaft inner diameter (ID) that is 17-35% of the balloon outer diameter (OD); a 12 mm balloon may have a shaft outer diameter (OD) that is 15-30% of the balloon outer diameter (OD); and a 12 mm balloon may have a shaft inner diameter that is 11-17% of the balloon outer diameter (OD).

The shaft ID % strain is calculated by the following formula:

$$\frac{\text{ID stretched tube} - \text{ID extruded tube}}{\text{ID extruded tube}}$$

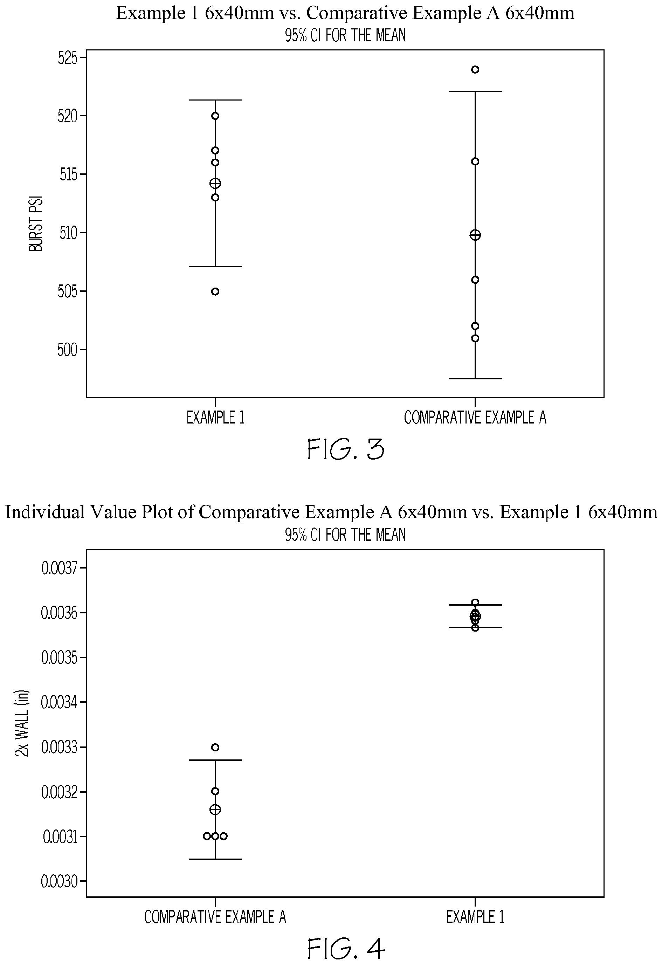

FIGS. 3-4 graphically compare an integral balloon shaft 22 with a 6 x 40 mm balloon 24 (Example 1), to a commercially available 2 layer balloon tacked or bonded onto a shaft (Comparative Example A). As can be seen, the burst pressures of the integral balloon 24 are comparable to the burst pressures of the tacked balloon of Comparative Example A; and the double wall thicknesses of the integral balloon 24 is slightly greater than the double wall thickness of the tacked balloon of Comparative Example A.

**Balloon Catheter**

In a further aspect, the integral balloon shaft 22 forms a part of a balloon catheter 20 that can be employed in any of a variety of medical procedures including, but not limited to angioplasty (PTCA) procedures; for delivery medical devices such as stents or valves; genitourinary procedures; biliary procedures; neurological procedures; peripheral vascular procedures; renal procedures; etc. The balloon catheter 20 is particularly useful for procedures that require high pressure to treat, such as a blockage of a major artery, at an AV Fistula vein or graft where resistant lesions occur as a result of hemodialysis, or where heavy calcification and arterial elasticity present extreme challenges to clinicians.

In addition to an integral balloon shaft 22, the balloon catheter 20 includes an inner shaft 26 positioned inside the balloon shaft 22 (e.g. FIG. 2). The inner shaft 26 has a length greater than the length of the balloon shaft 22, and an outer diameter less than the inner diameter of the balloon shaft 22. In one aspect, the inner shaft 26 is tubular and defines a guidewire lumen for passage of a guidewire 32 therethrough (e.g. FIGS. 1-2).

The inner shaft 26 comprises a polymer. The inner shaft can have a single polymeric layer or a plurality of polymeric layers. Any suitable polymer can be used for the inner shaft 26. Examples of suitable polymers for the inner shaft include, but are not limited to: high density polypropylene (e.g. Marlex®); poly(ether-block-amides) (e.g. Pebax®); polyamides (e.g. Grilamid®); and combinations thereof. Further, the inner shaft can have a reinforcement layer, as is known in the art.

A distal end region of the inner shaft 26 and a distal end region of the balloon shaft 22 can be secured to one another to form a distal tip 28 of the balloon catheter 20 (e.g. FIG. 2). Any suitable means can be used to secure the shafts 22, 26 to one another. The distal tip can be a tapered bumper tip. In one aspect, the guidewire lumen extends through the distal tip (e.g. FIGS. 1-2). In a further aspect, the distal tip 28 has a length of about 4 mm to about 5 mm. Thus, the distal end of the balloon 24 is proximal to the distal end of the balloon shaft 22, and is proximal to the distal end of the balloon catheter 20.

In a further aspect, a balloon catheter 20 as described herein includes a manifold/handle 30 (herein after

EP 3 142 738 B1

manifold) at the proximal end (e.g. FIG. 1). In one aspect, the manifold is secured to both the balloon shaft 22 and the inner shaft 26. The manifold 30 has a plurality of outlets 31. In one aspect, the manifold has two outlets with one outlet in communication with the inflation lumen of the balloon shaft 22, and another outlet in communication with the guidewire lumen of the inner shaft 26.

Thus, in one aspect, the balloon catheter 20 has three components: the balloon shaft 22 with integral balloon 24; an inner shaft 26; and a manifold 30.

The balloon catheter 20 may include one or more areas, bands, coatings, members, etc. that is (are) detectable by imaging modalities such as X-Ray, MRI, ultrasound, etc. In some embodiments at least a portion of the balloon catheter 20 is at least partially radiopaque.

In a further aspect, at least a portion of the balloon catheter 20 is configured to include one or more mechanisms for the delivery of a therapeutic agent. Often the agent will be in the form of a coating or other layer (or layers) of material placed on a surface of the balloon catheter 20. The surface with a coating or layer can be the outer surface, the inner surface, or both the inner and outer surfaces. In one aspect, the balloon 24 has a coating of therapeutic agent.

A therapeutic agent may be a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent may be a polystyrene-polyisobutylene-polystyrene triblock copolymer (SIBS), polyethylene oxide, silicone rubber and/or any other suitable substrate.

## Method

As discussed above, a balloon shaft 22 with an integral balloon 24 is formed from an extruded tube. In summary, to form the balloon shaft 22 with an integral balloon 24, the extruded tube is formed into a stretched extruded tube before a section of the extruded tube is formed into a balloon 24.

To stretch the extruded tube, the extruded tube can be placed into a stretching machine with two clamps, for example, one movable clamp and one stationary clamp. The movable clamp is attached to the distal end region of the extruded tube and the stationary clamp is attached to the proximal end region of the extruded tube. A gas pressure source is in communication with the lumen of the extruded tube. The stationary clamp may have rubber pads which fix the extruded tube in position, but allows gas flow to pressurize the extruded tube.

The extruded tube is subjected to a multistage stretching process having three stretching steps or stages. In the first stretching step, a selected portion of the distal end region of the extruded tube is stretched at a first pressure and a first temperature, where the first pressure is equal to ambient pressure. In the second stretching step, the extruded tube is stretched at a second pressure and the first temperature until the extruded tube is stretched to 25 to 75%, preferably, 35-65%, more preferably 45-55%, and most preferably 50% the desired final length, where the second pressure is greater than the first pressure. In the third stretching step, the extruded tube is stretched at a third pressure and the first temperature until the extruded tube is stretched to the final stretched length, where the third temperature is less than the second pressure and greater than the first pressure.

The first stretching step typically reduces the dimensions of the distal end region of the extruded tube, and the third stretching step advantageously optimizes the material modulus, the inner diameter, and the outer diameter of the stretched extruded tube.

After the third stretching step, the stretched extruded tube is quenched at a second temperature less than ambient temperature, and the pressure is reduced from the third pressure. Then a portion of the stretched extruded tube is placed into a balloon mold for formation of the integral balloon 24. The balloon mold may have three sections, a distal end section, a body section, and a proximal end section. While in the mold, the stretched tube is pressurized to a fourth pressure at an elevated third temperature to form the balloon 24, where the third temperature is greater than the first temperature; the balloon 24 is heat set at a fourth temperature greater than the third temperature; and then quenched at a reduced fourth temperature for removal of the balloon shaft 22 with an integral balloon 24 from the mold, where the fourth temperature is less than ambient temperature.

This manufacturing method is a simplified manufacturing process that achieves the same superior balloon properties as commercially available balloons. This method is described in greater detail with reference to Examples A-E.

**Stretching the Extruded Tube**

**Example A**

The extruded tube is placed into the two clamps of the stretching machine. An initial distance of 516 mm separates the two clamps.

For the first stretching step, a distal region of the extruded tube next to the movable clamp is contact heated to a first temperature, and the extruded tube is stretched to form a distal neck with transition. In one aspect, the first temperature is 80° C; the first pressure is ambient pressure; and the distal region is approximately 127 mm (5 inches).

For the second stretching step, the extruded tube is stretched to a distance of about 716 mm in an 80° C hot bath, at a velocity of 50 mm/sec, and an inflation pressure of 280 psi ($1.930532 \times 10^6$ Pa).

When the extruded tube reaches a length of about 716 mm, the third stretching step begins. In the third stretching step, the pressure is reduced to 180 psi ($1.241056 \times 10^6$ Pa) and the extruded tube is stretched to a final length.

After the third stretching step, the stretched extruded tube is quenched in a 11° C bath, the pressure is reduced from 180 psi ($1.241056 \times 10^6$ Pa), and the stretched extruded tube is released from the two clamps that are separated at least by 990 mm.

**Examples B-D**

The extruded tube is placed into the two clamps of the stretching machine. An initial distance of about 366 mm separates the two clamps.

For the first step, a distal region of the extruded tube next to the movable clamp is contact heated to a first temperature, and the extruded tube is stretched to form a distal neck with transition. In one aspect, the distal region has a length of about 25 mm; the first temperature is 80° C; the pressure is ambient pressure; and the extruded tube is stretched a distance of about 416 mm.

For the second stretching step, the extruded tube is stretched in an 80° C hot bath at a velocity of 50 mm/sec to a distance of about 716 mm at an inflation pressure of 357 psi ($2.461428 \times 10^6$ Pa).

When the extruded tube reaches a length of about 716 mm, the third stretching step begins. In the third stretching step, the pressure is reduced to 290 psi ($1.9994796 \times 10^6$ Pa) and stretched to about 990 mm.

Then the stretched extruded tube is quenched in a 11° C bath, the pressure is reduced from 290 psi ($1.9994796 \times 10^6$ Pa), and the stretched extruded tube released from the two clamps that separated at least by 990 mm.

**Example E**

The extruded tube is placed into the two clamps of the stretching machine. An initial distance of about 336 mm separates the two clamps.

For the first step, a distal region of the extruded tube next to the movable clamp is contact heated to a first temperature, and the extruded tube is stretched to form a distal neck with transition. In one aspect, the distal region has a length of about 25 mm; the first temperature is 80° C; the pressure is ambient pressure; and the extruded tube is stretched a distance of about 386 mm.

For the second stretching step, the extruded tube is stretched in an 80° C hot bath at a velocity of 50 mm/sec to a distance of about 716 mm at an inflation pressure of 275 psi ($1.896058 \times 10^6$ Pa).

When the extruded tube reaches a length of about 716 mm, the third stretching step begins. In the third stretching step, the pressure is reduced to 190 psi ($1.310039 \times 10^6$ Pa), and the extruded tube is stretched to about 990 mm.

Then the stretched tube is quenched in an 11° C bath, the pressure is reduced from 190 psi ($1.310039 \times 10^6$ Pa), and the stretched tube released from the two clamps that are separated by 990 mm.

Aspects of the stretched tube used to form the integral balloon shaft of Examples A-E are provided in Table 5:

**Table 5**

| | Stretched Tube Dimensions | | | | |
|---|---|---|---|---|---|
| | End Section | | Shaft | | Total |
| Ex. | OD (mm) | ID (mm) | OD (mm) | ID (mm) | Length (cm) |
| A | 0.029 | 0.018 | 0.067 | 0.058 | 79 |
| B | 0.069 | 0.060 | 0.069 | 0.060 | 79 |
| C | 0.069 | 0.060 | 0.069 | 0.060 | 79 |

(continued)

| | Stretched Tube Dimensions | | | | |
|---|---|---|---|---|---|
| | End Section | | Shaft | | Total |
| Ex. | OD (mm) | ID (mm) | OD (mm) | ID (mm) | Length (cm) |
| D | 0.069 | 0.060 | 0.069 | 0.060 | 79 |
| E | 0.081 | 0.065 | 0.081 | 0.065 | 79 |

**Forming the Integral Balloon**

In one aspect, a balloon mold with a 50.8 mm (2 inches) distal end section, a 40 mm body section, and a 101.6 mm (4 inches) proximal end section is used to form a 40 mm balloon. The transition between the smaller outer diameter distal end region and larger outer diameter proximal end region of the stretched tube is placed in the proximal mold section. The stretched tube and mold are placed into a mold holder and then onto the molding machine arm. The stretched tube is pressurized at a fourth pressure and placed in bath at a third temperature until the balloon is formed. Depending on the diameter of the balloon to be formed, the fourth pressure may be less than or greater than the third pressure.
Next, the mold is transferred to a heat set station set at a fourth temperature for 60-90 seconds. In one aspect, the fourth temperature is greater than the first temperature.
Then the mold is transferred to a quench bath at a fifth temperature to cool the shaft and mold to allow for removal of the balloon shaft 22 with the integral balloon 24 from the mold. In one aspect the fifth temperature, less than the first temperature

**Example A**

To form a 3 x 40 mm balloon, the stretched tube in the balloon mold is pressurized with nitrogen gas at 500 psi (3.4473786x10$^6$ Pa) and placed in a 95° C bath until the balloon is formed. Next, the mold is transferred to a heat set station set for 125° C for one (1) minute before the mold is transferred to a 10° C quench bath to cool the shaft and mold to allow for removal of the balloon shaft 22 with the integral balloon 24 from the mold.

**Examples B-D**

To form a 7 x 40 mm balloon, the stretched tube in the balloon mold is pressurized with nitrogen gas at 265 psi (1.827111x10$^6$ Pa) and placed in a 95° C bath until the balloon is formed. Next, the mold is transferred to a heat set station set for 125° C for one (1) minute before the mold is transferred to a 10° C quench bath to cool the shaft and mold to allow for removal of the balloon shaft 22 with the integral balloon 24 from the mold.

**Example E**

To form a 12 x 40 mm balloon, the stretched tube in the balloon mold is pressurized with nitrogen gas at 300 psi (2.068427x10$^6$ Pa) and placed in a 95° C bath until the balloon is formed. Next, the mold is transferred to a heat set station set for 125° C for one (1) minute before the mold is transferred to a 10° C quench bath to cool the shaft and mold to allow for removal of the balloon shaft 22 with the integral balloon 24 from the mold.
Aspects of forming the integral balloon of Examples A-E are provided in Table 6.

**Table 6**

| Ex. | Balloon Mold ID (mm) | Balloon ID Blow Ratio |
|---|---|---|
| A | 3.0099 | 5.56 |
| B | 6.8834 | 6.20 |
| C | 6.8834 | 6.00 |
| D | 6.8834 | 6.00 |
| E | 11.8364 | 6.60 |

**Forming a Balloon Catheter with the Integral Balloon Shaft**

As noted above, a balloon shaft 22 with integral balloon 24 can form a part of a balloon catheter 20. In one aspect, a method of forming a balloon catheter 20 includes: inserting a polymeric tube into the lumen of a shaft 22 with the integral balloon 24, where the shaft 22 is considered to be an outer shaft and the polymeric tube is considered to be an inner shaft 26; securing the outer shaft 22 and inner shaft 26 to one another to form a shaft assembly; and attaching a manifold 30 to a proximal end of the shaft assembly to form the balloon catheter 20.

Any suitable means can be used to secure the outer shaft 22 and inner shaft 26. For example, a hot jaw, a laser bonder, a means of fusion, or a means of adhesion can be used to fuse the outer shaft 22 and inner shaft 26. In one aspect, securing the outer shaft 22 and inner shaft 26 to one another forms a distal tip 28, such as a tapered bumper tip. In a further aspect, about 4-5 mm of the outer shaft 22 distal to the balloon 24 is fused to the inner shaft 26.

Any suitable means can be used to attach the manifold 30 to the shaft assembly. For example, a UV cure adhesive; a two part epoxy; a high strength adhesive; or any other means of adhesion can be used to attach the manifold. As used herein a "high strength adhesive" is an adhesive capable of withstanding internal pressure of 500 psi ($3.447379 \times 10^6$ Pa) and higher.

The above disclosure is intended to be illustrative and not exhaustive. This description will suggest many variations and alternatives to one of ordinary skill in this art. The various elements shown in the individual figures and described above may be combined or modified for combination as desired. All these alternatives and variations are intended to be included within the scope of the claims where the term "comprising" means "including, but not limited to".

The invention is defined by the appended claims.

**Claims**

1.   A method of forming a multilayer shaft (22), comprising:

   a first stretching step wherein a portion of a distal end region of a multilayer polymeric tube is stretched at a first pressure and a first temperature, and the first pressure is equal to ambient pressure;
   a second stretching step wherein the multilayer polymeric tube is stretched at a second pressure and the first temperature until the extruded tube is stretched to 25 to 75%, preferably, 35-65%, more preferably 45-55%, and most preferably 50% of a desired final length, and the second pressure is greater than the first pressure;
   a third stretching step wherein the multilayer polymeric tube is stretched at a third pressure and the first temperature to form a stretched multilayer polymeric tube having the final stretched length, and the third pressure is less than the second pressure and greater than the first pressure; and
   a balloon forming step wherein a section of the stretched multilayer polymeric tube is formed into an integral balloon (24),
   wherein a total shaft length of the multilayer shaft (22) with the integral balloon (24) is at least 70 cm.

2.   The method of claim 1, wherein forming the integral balloon (24) comprises:

   placing the section of the stretched multilayer polymeric tube into a balloon mold;
   pressurizing the stretched multilayer polymeric tube to a fourth pressure at a second temperature to form the section into the integral balloon (24), wherein the fourth pressure is different than the first, second, and third pressures, and the second temperature is greater than the first temperature; and
   heat setting the integral balloon (24) at a third temperature greater than the second temperature.

3.   The method of any one of claims 1-2, further comprising a first quenching step after the third stretching step, wherein during the first quenching step the temperature is reduced to a fourth temperature less than the first temperature and pressure is reduced from the third pressure.

4.   The method of claim 3, the method further comprising a second quenching step after the heat setting step, wherein during the second quenching step the temperature is reduced to a fifth temperature less than the first temperature; further wherein the integral balloon (24) is removed from the mold after the second quenching step.

5.   The method of any one of claims 1-4, wherein the multilayer polymeric tube is formed by coextrusion.

6.   The method of claim 1, wherein the integral balloon (24) has a burst strength of at least 30,000 psi ($3.068427 \times 10^8$ Pa).

7. The method of any one of claims 1 and 6, wherein the integral balloon (24) has a distension per atmosphere between 60.7950*10$^4$ and 1.419*10$^6$ N/m$^2$ (6 and 14 atmospheres) that is no greater than 0.9%.

8. The method of any one of claims 1, 6 and 7, wherein the integral balloon (24) has a double wall thickness of 0.0254 mm to 0.127 mm.

9. The method of any one of claims 1 and 6 to 8, wherein the integral balloon (24) has a balloon outer diameter of 1.5 mm to 28 mm.

10. The method of any one of claims 1 and 6 to 9, wherein the shaft (22) has a shaft outer diameter that is 15-55% of the balloon outer diameter.

11. The method of any one of claims 1 and 6 to 10, wherein the shaft (22) has a shaft inner diameter that is 11-50% of the balloon outer diameter.

12. The method of any one of claims 1 and 6 to 11, wherein the shaft (22) has an outer layer formed of nylon and an inner layer formed of poly(ether-block-amide).

13. The method of any one of claims 1 and 6 to 12, wherein the shaft (22) has an inner layer formed of poly(ether-block-amide), a middle layer formed of nylon, and an outer layer formed of poly(ether-block-amide).

14. The method of any one of claims 12 and 13, wherein the nylon is selected from the group consisting of nylon 12; nylon 6; nylon 6/10; nylon 6/12; nylon 11; and aromatic nylons; and the poly(ether-block-amide) has a shore D hardness of 60-74D.


**Patentansprüche**

1. Verfahren zum Bilden eines mehrlagigen Schafts (22), das aufweist:

   einen ersten Dehnungsschritt, bei dem ein Teil eines distalen Endbereichs eines mehrlagigen Polymerschlauchs bei einem ersten Druck und einer ersten Temperatur gedehnt wird und der erste Druck gleich dem Umgebungsdruck ist;
   einen zweiten Dehnungsschritt, bei dem der mehrlagige Polymerschlauch bei einem zweiten Druck und der ersten Temperatur gedehnt wird, bis der extrudierte Schlauch auf 25 bis 75 %, bevorzugt 35-65 %, noch bevorzugter 45-55 % und am meisten bevorzugt 50 % einer gewünschten Endlänge gedehnt ist, und der zweite Druck größer als der erste Druck ist;
   einen dritten Dehnungsschritt, bei dem der mehrlagige Polymerschlauch bei einem dritten Druck und der ersten Temperatur gedehnt wird, um einen gedehnten mehrlagigen Polymerschlauch zu bilden, der die gedehnte Endlänge aufweist, und der dritte Druck kleiner als der zweite Druck und größer als der erste Druck ist; und
   einen Ballonformungsschritt, bei dem ein Abschnitt des gedehnten mehrlagigen Polymerschlauchs zu einem einstückigen Ballon (24) geformt wird,
   wobei eine Gesamtschaftlänge des mehrlagigen Schafts (22) mit dem einstückigen Ballon (24) mindestens 70 cm beträgt.

2. Verfahren nach Anspruch 1, wobei das Formen des einstückigen Ballons (24) aufweist:

   Einlegen des Abschnitts des gedehnten mehrlagigen Polymerschlauchs in eine Ballonform;
   Beaufschlagen des gedehnten mehrlagigen Polymerschlauchs mit einem vierten Druck bei einer zweiten Temperatur, um den Abschnitt zu dem einstückigen Ballon (24) zu formen, wobei der vierte Druck vom ersten, zweiten und dritten Druck verschieden ist und die zweite Temperatur höher als die erste Temperatur ist; und
   Thermofixieren des einstückigen Ballons (24) bei einer dritten Temperatur, die höher als die zweite Temperatur ist.

3. Verfahren nach einem der Ansprüche 1-2, ferner aufweisend einen ersten Abschreckschritt nach dem dritten Dehnungsschritt, wobei die Temperatur beim ersten Abschreckschritt auf eine vierte Temperatur gesenkt wird, die niedriger als die erste Temperatur ist, und der Druck vom dritten Druck aus gesenkt wird.

**4.** Verfahren nach Anspruch 3, wobei das Verfahren ferner einen zweiten Abschreckschritt nach dem Thermofixierschritt aufweist, wobei bei dem zweiten Abschreckschritt die Temperatur auf eine fünfte Temperatur gesenkt wird, die niedriger als die erste Temperatur ist; wobei ferner der einstückige Ballon (24) nach dem zweiten Abschreckschritt aus der Form entnommen wird.

**5.** Verfahren nach einem der Ansprüche 1-4, wobei der mehrlagige Polymerschlauch durch Koextrudieren gebildet wird.

**6.** Verfahren nach Anspruch 1, wobei der einstückige Ballon (24) eine Berstdruckfestigkeit von mindestens 30.000 psi (3,068427x10$^8$ Pa) aufweist.

**7.** Verfahren nach einem der Ansprüche 1 und 6, wobei der einstückige Ballon (24) pro Atmosphäre eine Ausdehnung zwischen 60,7950 * 10$^4$ und 1,419 * 10$^6$ N/m$^2$ (6 und 14 Atmosphären) aufweist, die nicht größer als 0,9 % ist.

**8.** Verfahren nach einem der Ansprüche 1, 6 und 7, wobei der einstückige Ballon (24) eine Doppelwanddicke von 0,0254 mm bis 0,127 mm aufweist.

**9.** Verfahren nach einem der Ansprüche 1 und 6 bis 8, wobei der einstückige Ballon (24) einen Ballonaußendurchmesser von 1,5 mm bis 28 mm aufweist.

**10.** Verfahren nach einem der Ansprüche 1 und 6 bis 9, wobei der Schaft (22) einen Schaftaußendurchmesser von 15-55 % des Ballonaußendurchmessers aufweist.

**11.** Verfahren nach einem der Ansprüche 1 und 6 bis 10, wobei der Schaft (22) einen Schaftinnendurchmesser von 11-50 % des Ballonaußendurchmessers aufweist.

**12.** Verfahren nach einem der Ansprüche 1 und 6 bis 11, wobei der Schaft (22) eine aus Nylon gebildete Außenlage und eine aus Poly(ether-Block)-Amid gebildete Innenlage aufweist.

**13.** Verfahren nach einem der Ansprüche 1 und 6 bis 12, wobei der Schaft (22) eine aus Poly(ether-Block)-Amid gebildete Innenlage, eine aus Nylon gebildete Mittellage und eine aus Poly(ether-Block)-Amid gebildete Außenlage aufweist.

**14.** Verfahren nach einem der Ansprüche 12 und 13, wobei das Nylon aus der Gruppe bestehend aus Nylon 12, Nylon 6, Nylon 6/10, Nylon 6/12, Nylon 11 und aromatischen Nylons ausgewählt ist und das Poly(ether-Block)-Amid eine Shore D-Härte von 60-74D aufweist.

**Revendications**

**1.** Procédé de formation d'une tige multicouche (22), comprenant :

une première étape d'étirage dans laquelle une partie d'une région d'extrémité distale d'un tube polymère multicouche est étirée sous une première pression et à une première température, et la première pression est égale à la pression ambiante ;
une deuxième étape d'étirage dans laquelle le tube polymère multicouche est étiré sous une deuxième pression et à la première température jusqu'à ce que le tube extrudé soit étiré à 25 à 75 %, de préférence à 35 à 65 %, mieux encore à 45 à 55 %, et tout spécialement à 50 % de la longueur finale souhaitée, et la deuxième pression est supérieure à la première pression ;
une troisième étape d'étirage dans laquelle le tube polymère multicouche est étiré sous une troisième pression et à la première température pour former un tube polymère multicouche étiré ayant la longueur étirée finale, et la troisième pression est inférieure à la deuxième pression et supérieure à la première pression ; et
une étape de formation de ballonnet dans laquelle une section du tube polymère multicouche étiré est mis sous la forme d'un ballonnet monobloc (24),
dans lequel la longueur de tige totale de la tige multicouche (22) avec le ballonnet monobloc (24) est d'au moins 70 cm.

**2.** Procédé selon la revendication 1, dans lequel la formation du ballonnet monobloc (24) comprend :

le placement de la section du tube polymère multicouche étiré dans un moule de ballonnet ;

la pressurisation du tube polymère multicouche étiré sous une quatrième pression à une deuxième température pour mettre la section sous la forme du ballonnet monobloc (24), dans laquelle la quatrième pression est différente des première, deuxième et troisième pressions, et la deuxième température est supérieure à la première température ; et

la stabilisation à la chaleur du ballonnet monobloc (24) à une troisième température supérieure à la deuxième température.

3. Procédé selon l'une quelconque des revendications 1 et 2, comprenant en outre une première étape de trempe après la troisième étape d'étirage, dans lequel, durant la première étape de trempe, la température est réduite à une quatrième température inférieure à la première température et la pression est réduite par rapport à la troisième pression.

4. Procédé selon la revendication 3, lequel procédé comprend en outre une deuxième étape de trempe après l'étape de stabilisation à la chaleur, dans lequel, durant la deuxième étape de trempe, la température est réduite à une cinquième température inférieure à la première température ;

dans lequel en outre le ballonnet monobloc (24) est retiré du moule après la deuxième étape de trempe.

5. Procédé selon l'une quelconque des revendications 1 à 4, dans lequel le tube polymère multicouche est formé par coextrusion.

6. Procédé selon la revendication 1, dans lequel le ballonnet monobloc (24) a une résistance à l'éclatement d'au moins 30 000 psi (3,068427 x $10^8$ Pa).

7. Procédé selon l'une quelconque des revendications 1 et 6, dans lequel le ballonnet monobloc (24) a une distension par atmosphère entre 60,7950*$10^4$ et 1,419*$10^6$ N/m$^2$ (entre 6 et 14 atmosphères) qui n'est pas supérieure à 0,9 %.

8. Procédé selon l'une quelconque des revendications 1, 6 et 7, dans lequel le ballonnet monobloc (24) a une épaisseur de double paroi de 0,0254 mm à 0,127 mm.

9. Procédé selon l'une quelconque des revendications 1 et 6 à 8, dans lequel le ballonnet monobloc (24) a un diamètre extérieur de ballonnet de 1,5 mm à 28 mm.

10. Procédé selon l'une quelconque des revendications 1 et 6 à 9, dans lequel la tige (22) a un diamètre extérieur de tige qui est de 15 à 55 % du diamètre extérieur de ballonnet.

11. Procédé selon l'une quelconque des revendications 1 et 6 à 10, dans lequel la tige (22) a un diamètre intérieur d'arbre qui est de 11 à 50 % du diamètre extérieur de ballonnet.

12. Procédé selon l'une quelconque des revendications 1 et 6 à 11, dans lequel la tige (22) a une couche extérieure formée de nylon et une couche intérieure formée de poly(éther-bloc-amide).

13. Procédé selon l'une quelconque des revendications 1 et 6 à 12, dans lequel la tige (22) a une couche intérieure formée de poly(éther-bloc-amide), une couche centrale formée de nylon, et une couche extérieure formée de poly(éther-bloc-amide).

14. Procédé selon l'une quelconque des revendications 12 et 13, dans lequel le nylon est choisi dans le groupe constitué par le nylon 12 ; le nylon 6 ; le nylon 6/10 ; le nylon 6/12 ; le nylon 11 ; et les nylons aromatiques ; et le poly(éther-bloc-amide) a une dureté Shore D de 60 à 74 D.

FIG.1

FIG. 2

Example 1 6x40mm vs. Comparative Example A 6x40mm
95% CI FOR THE MEAN

FIG. 3

Individual Value Plot of Comparative Example A 6x40mm vs. Example 1 6x40mm
95% CI FOR THE MEAN

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010130926 A1
- US 2009198266 A1
- EP 1100573 A1
- EP 0921832 A1